# EUROPEAN PATENT SPECIFICATION

(11) **EP 2 964 751 B1**
(45) Date of publication and mention of the grant of the patent: **20.08.2025**
(21) Application number: 14712921.7
(22) Date of filing: 05.03.2014
(51) Int. Cl.: C12N 7/00

(54) **PRODUCTION OF AVIAN EMBRYO CELLS**
HERSTELLUNG VON VOGELEMBRYOZELLEN
PRODUCTION DE CELLULES EMBRYONNAIRES AVIAIRES

(30) Priority: 05.03.2013 US 201313785513
(43) Date of publication of application: 13.01.2016
(73) Proprietor: Biomune Company, Lenexa, KS 66215 (US)
(72) Inventor: SAWARKAR, Sharad, Devidasrao, Overland Park, KS 66210 (US); GULLY, Christopher, Patrick, Shawnee, KS 66216 (US)
(74) Representative: Cabinet Becker et Associés
(86) International application number: PCT/US2014/020597
(87) International publication number: WO 2014/138180

(56) References cited:
- EP-A1- 1 500 699
- WO-A1-03/055988
- WO-A1-03/076601
- WO-A1-2005/007840
- WO-A1-2005/068610
- WO-A1-2008/017704
- WO-A2-2005/042728
- FR-A1- 2 884 255
- US-A- 5 656 479
- US-A1- 2005 149 996
- EYAL-GILADI H ET AL: "From cleavage to primitive streak formation: A complementary normal table and a new look at the first stages of the development of the chick - I. General morphology", DEVELOPMENTAL BIOLOGY, ACADEMIC PRESS, AMSTERDAM, NL, vol. 49, no. 2, 1 April 1976 (1976-04-01), pages 321 - 337, XP024780591, ISSN: 0012-1606, [retrieved on 19760401], DOI: 10.1016/0012-1606(76)90178-0
- KOCHAV S ET AL: "From cleavage to primitive streak formation: A complementary normal table and a new look at the first stages of the development of the chick - II. Microscopic anatomy and cell population dynamics", DEVELOPMENTAL BIOLOGY, ACADEMIC PRESS, AMSTERDAM, NL, vol. 79, no. 2, 1 October 1980 (1980-10-01), pages 296 - 308, XP024848810, ISSN: 0012-1606, [retrieved on 19801001], DOI: 10.1016/0012-1606(80)90117-7
- YANI ZHANG ET AL: "Isolation of chicken embryonic stem cell and preparation of chicken chimeric model", MOLECULAR BIOLOGY REPORTS, vol. 40, no. 3, 1 March 2013 (2013-03-01), pages 2149 - 2156, XP055121366, ISSN: 0301-4851, DOI: 10.1007/s11033-012-2274-8
- SHARON BOAST ET AL: "Simple methods for generating neural, bone and endodermal cell types from chick embryonic stem cells", STEM CELL RESEARCH, vol. 10, no. 1, 1 January 2013 (2013-01-01), pages 20 - 28, XP055121413, ISSN: 1873-5061, DOI: 10.1016/j.scr.2012.08.008

## Description

The present invention relates to compositions and methods for preparing cells from avian embryos. The invention also relates to cultures of such cells and the uses thereof, particularly for producing viruses such as MDV. The invention also relates to methods for the preparation of vaccines using such cells or viruses.

### BACKGROUND

Fertile avian eggs, such as chicken eggs, are one of the main substrates for the manufacture of human and veterinary vaccines. They have been used because they are able to support the replication of a wide range of human and animal viruses, including replication-defective viruses. Examples of vaccines produced in avian embryo cells include, for instance, MDV vaccine, avian Reovirus vaccine, and infectious bursal disease virus vaccine.

Vaccine production in avian embryo cells requires a continuous and reliable supply of fertile eggs from specified pathogen free (SPF) flocks. SPF flocks are raised under special conditions and are regularly demonstrated to be free of avian pathogens (D. H. Thornton, "Marek's Diseases: Scientific Basis and Methods of Control: quality control and standardization of vaccines." (L. N. Payne, Ed.), Martinus Nijoff Publishing, Boston, MA, pp. 267-292 (1985)). Embryos are obtained from the eggs, beheaded, and then treated (typically by mechanical and/or enzymatic treatments) to produce primary embryo cells, generally embryo fibroblasts. Typically, the embryos are separated from the head and are collected in a beaker. The bodies-only embryos are then transferred to a trypsinizing flask where they are trypsinized 3 times for ten minutes each in cold trypsin (colder than room temperature). The digestive buffer solution, containing the separated cells, is poured through a sieve (1 mm mesh size) and into a carboy containing serum. The collected CEF are concentrated by centrifuge and chilled until use.

Such cells can then be cultured and used to produce viruses.

As an example, current Marek's disease vaccines are either suspensions of infected Chicken Embryo Cells (CEF) or cell-free virus suspensions made from sonicated CEF infected with vaccine strains of Marek's disease virus (A. E. Churchill, "Marek's Diseases: Scientific Basis and Methods of Control: Production of vaccines" (L. N. Payne, Ed.), Martinus Nijoff Publishing, Boston, MA, pp. 251-266 (1985)).

As all primary animal cells, avian embryo fibroblasts undergo senescence, the doubling time increasing from passage to passage until the cells eventually die. Primary avian embryo cells such as chicken embryo cells (CEFs) have a finite life span of approximately two to three weeks. Accordingly, in order to sustain vaccine production, such cells must be prepared every one or two weeks, which increases the costs for producing vaccines.

In order to overcome the need for primary cells and the use of fertile eggs, attempts have been made to develop immortalized avian cell lines (see e.g., K. Nazerian, Avian Pathol. 16:527-544 (1987)). However, these cell lines derive from virally transformed cells, or produce tumors when inoculated into chickens, or generate insufficient titer of virus for commercial production. Accordingly, these cell lines cannot substitute for primary avian embryo fibroblast cells in vaccine production.

The present invention provides an alternative approach to improve avian embryo cell production suitable for vaccine production. The present invention discloses an improved method for producing primary avian cells suitable for vaccination from whole embryos. The method improves the number of cells generated and therefore reduces the cost of vaccine production.

### SUMMARY OF THE INVENTION

The present invention relates to improved methods for producing primary avian embryo cells. The invention also relates to such cells and the uses thereof to produce vaccines or other biological products.

The invention stems, inter alia, from the development of a novel method for producing cells from whole embryos. The results presented show that such method provides improved number of cells per embryo, and that such cells exhibit suitable characteristics (density, confluency, virus production titers) for commercial vaccine production. The results presented more particularly show that the method produces a CEF/embryo yield that is approximately double than what is achieved in the prior art, and allows seeding cells at a higher density with a harvest at approximately 50 hours post infection to achieve titers above minimum release. Such method therefore provides a substantial advantage as compared to prior art techniques which use established cell lines or conventional bodies-only embryo cells.

The present invention is as defined in the claims.

An object of the invention therefore resides in a method for preparing avian embryo cells comprising:
a) obtaining whole avian embryos from avian eggs, each of said whole avian embryos having a head and eyes, and
b) placing said whole avian embryos into one or several suitable recipients, such that each of said recipients contain between 50 and 150 whole avian embryos,
c) preparing avian embryos cells by enzymatic treatment of the whole embryo at a temperature comprised between 20°C and 40°C, and
d) filtering the cells with a filter having a pore size of between 20 microns and 50 microns.

In particular embodiments of the methods of the invention, the avian embryo cells may be cultured or expanded prior to or after filtration step d); and/or the avian embryo cells may be infected by a virus prior to or after filtration step d).

The avian egg is preferably a Specified Pathogen Free avian egg. Also, the avian embryo cells are preferably fibroblasts. The invention may be applied to any avian, preferably a chicken or duck. After production, the cells may be maintained in culture, preferably in a serum-free culture medium.

A further object of the invention relates to a culture or composition of avian embryo cells obtainable or obtained by a method as disclosed above, or progenitors of said cells.

Preferably, the cells are chicken embryo fibroblasts, optionally infected by a virus such as a Marek's disease virus (MDV).

A further object of the invention relates to a method for producing or amplifying a virus, comprising infecting cells as defined above with a virus and, optionally, collecting the virus produced. The virus produced or amplified may be subsequently inactivated. The virus may be any animal or human virus such as, without limitation, a MDV, Herpes Virus of Turkeys, MDV (SB-1), or MDV (Rispens), avian Reovirus and infectious bursal disease virus.

The invention also relates to a method for producing a viral vaccine comprising (i) infecting cells as defined above with a virus under conditions allowing production or amplification of the virus, (ii) optionally collecting the virus produced or amplified, (iii) optionally inactivating the virus produced or amplified in (i) or (ii), and (iv) formulating the virus produced or amplified as a vaccine composition.

In a particular embodiment of the method the virus produced or amplified is collected, isolated, filtered at 20 microns or more, optionally inactivated, and formulated as a vaccine composition.

According to another particular embodiment, the cells are filtered at 20 microns or more prior to step (i) and the cell supernatant or cell debris are used to produce the vaccine.

The invention also relates to a vaccine composition comprising a virus or cell as defined above or obtained by a method as defined above, and a suitable excipient or carrier.

The invention is particularly suited to produce CEF and to use such cells for production of a MDV vaccine.

### LEGEND TO THE FIGURES

The patent or application file contains at least one drawing executed in color. Copies of this patent or patent application publication, with color drawing(s), will be provided by the Office upon request and payment of the necessary fee.
Figure 1: Survival study of CEF prepared from whole embryos.
Figure 2: Confluency study of CEF prepared from whole embryos.
Figure 3: Marek's growth on CEF cells from whole embryos.

### DETAILED DESCRIPTION OF THE INVENTION

The present invention relates to compositions and methods for preparing cells from avian embryos. The invention also relates to cultures of such cells and the uses thereof, particularly for producing viruses such as MDV. The invention also relates to methods for the preparation of vaccines using such cells or viruses.

### Definitions

Within the context of the invention, the term "avian" includes, without limitation, chicken, duck, goose, and birds. A preferred avian is chicken.

The term "whole" or "entire" embryo designates an embryo which is not treated to remove any part thereof. In particular, a whole embryo contains a head (as opposed to bodies-only embryos) and eyes.

MDV designates any of the three MDV serotypes, i.e., serotype 1, serotype 2, or serotype 3. Serotype 3 is also referred to as turkey herpesvirus (HVT). The MDV can be a recombinant virus comprising foreign genes or a deletion mutant which is used as a vaccine or for other uses. The MDV can also be a defective virus comprising an origin of replication and foreign DNA sequences.

### Preparation of avian embryo cells

A first aspect of the invention relates to an improved method for producing avian embryo cells based on the use of whole embryos.

In the prior art, methods of generating embryo cells always used bodies-only embryos. Indeed, the head portion was believed to contain non-relevant cell populations and to contaminate the cells with pigments, especially pigmented epithelial cells from the eyes.

Our results surprisingly show that, by using whole embryos, it is possible to increase the yield of cells per embryo by 50% and more. Such an increase provides a very substantial reduction of the overall cost of vaccines produced in embryo cells. Furthermore, the whole embryo CEF preparation method also cuts the labor requisite thus helping to decrease the manipulation and risk of contamination. The results obtained further unexpectedly show that embryo cells prepared with whole embryos are a suitable substrate for vaccine production. In particular, we compared whole embryos versus embryo bodies in several aspects of cell and virus growth and showed that the methods of the present invention can generate cells with remarkable survival rate in different plant densities, as well as suitable sensitivity and virus proliferation time course.

An object of the invention therefore relates to a method for preparing avian embryo cells as defined in claim 1, of which the key steps are:
obtaining whole avian embryos from avian eggs,
preparing cells from the entire embryos, and
filtering the cell preparation.

These steps are described below. The improvement resides in preparing the cells from the entire embryo and subsequently filtering the cell preparation.

Avian eggs may be obtained from any commercial source known in the art. Indeed, such eggs are already commonly used to generate embryo cells. Preferably, the invention uses specified pathogen free (SPF) eggs. SPF flocks are raised under special conditions and are regularly demonstrated to be free of avian pathogens (D. H. Thornton, "Marek's Diseases: Scientific Basis and Methods of Control: quality control and standardization of vaccines." (L. N. Payne, Ed.), Martinus Nijoff Publishing, Boston, MA, pp. 267-292 (1985)). The use of SPF eggs ensures the lack of contamination of the embryo cells and any biological product produced from such cells.

Embryos are obtained from the eggs and collected in a suitable recipient, such as a beaker, flask, bottle, or the like. As specified, the invention utilizes whole embryos, i.e., embryos which are not treated to remove the head, the eyes, or any other portion. Usually, from 50 to 800 whole embryos are collected and placed in the same recipient for subsequent preparation of the cells.

Cells can be prepared from the whole embryos according to techniques known per se in the art. More particularly, the cells can be prepared by mechanical and/or enzymatic digestion of the whole embryos, resulting in a dissociation of the tissues into separated cells. In a particular embodiment, the whole embryos are trypsinized, 1 to 3 times for five to twenty minutes each. The digestive buffer solution, containing the separated cells, is then poured through a sieve (1 mm mesh size) and into a carboy containing serum (5-20 % of the final volume).

During this preparation, trypsinization is usually conducted in cold trypsin, i.e., colder than room temperature. Surprisingly, the inventors have now shown that an improved yield is obtained if the preparation is performed with warm trypsin. In a particular embodiment, the cells are therefore prepared from the whole embryos by trypsinization at room temperature (20-25 degrees Celsius), or above.

In the method of the invention for preparing avian embryo cells, the embryos are submitted to an enzymatic digestion at a temperature comprised between 20 and 40°C, preferably between 20 and 25°C such as 20, 21, 22, 23, 24 or 25°C. Alternatively, digestion may be performed at higher temperatures comprised between 30°C and 40°C, such as 34, 35, 36, 37, 38 or 39°C. Digestion is preferably performed with trypsin, typically for 5 to 20 minutes, and may be repeated several times.

The inventors have also discovered that the performance of the method is improved by controlling the number of embryos used for each preparation. More particularly, the results obtained show that by using not more than approximately 150 embryos in one single preparation lot, it is possible to optimize the number of cells obtained per embryo.

In the method of the invention for preparing avian embryo cells, the embryos are placed into one or several suitable recipients, wherein each of said at least one recipients contains between approximately 50 and 150 embryos.

The decrease in tissue to volume ratio, coupled with increasing the trypsin activity by raising the temperature, allowed to more efficiently separate the whole embryos into individual cells and to reduce the left over material that is caught by the sieve and which does not go into the roller bottle. Also, the control of the number of embryos per recipient allowed to further improve the cell/embryo ratio.

With this method we were able to achieve a yield of greater than 6E+08 cells per embryo on a batch of 600 eggs. Marek's preparations of embryos with prior art techniques typically yield 2.0-2.5E+08 cells per embryo. Our results therefore demonstrate that the invention allows to significantly increase the yield per embryo, from 200% to 300%.

The collected avian embryo cells may then be concentrated (e.g., by centrifuge), cultured, expanded, and/or frozen until use.

In the method of the invention for preparing avian embryo cells, filtering the cells can be performed with a sieve diameter of between 20 and 50 microns. The use of whole embryos leads to a cell preparation containing pigmented epithelial cells from the retina. Such cells may introduce black spots in the preparation. Preferably, such cells are eliminated from the preparation by filtration, typically at 20 microns or more. The invention shows that, under such filtration conditions, these cells are completely separated so they are macroscopically invisible. Filtration may be performed using various techniques or filters known per se in the art, such as Sartorius polypropylene PP cartridge of 50 micron pore size at a flow rate of, for instance, 1 Lpm, or a blackflush system. By using a 50-70 microns system, it is possible to remove all visible black spots without losing any substantial amount of fibroblasts embryo cells.

Filtration may be performed on the embryo cell preparation prior to or after any subsequent treatment such as concentration, expansion, culture, infection, etc.

In a particular embodiment, filtration is performed on the concentrated embryo cells prior to addition to tissue culture medium.

In another particular embodiment, the avian embryo cells are cultured or expanded prior to or after filtration step.

Still in a further particular embodiment, the avian embryo cells are infected by a virus prior to or after filtration step.

Accordingly, the method of the invention for preparing avian embryo cells comprises:
- obtaining whole avian embryos, as defined herein,
- placing said whole avian embryos into one or several suitable recipients, wherein each of said one or several recipients contains between approximately 50 and 150 whole avian embryos,
- submitting the whole avian embryos in said recipients to enzymatic digestion at a temperature comprised between approximately 20 and 40°C, and
- filtering the cells with a filter having a pore size of between 20 microns and 50 microns.

As indicated, in a preferred embodiment, the avian embryo cells are fibroblasts. This may be validated by visual observation, or by testing for a number of fibroblast-specific features or markers. Fibroblasts are the fastest growing primary cells from avian species. When a cell suspension from whole avian embryos is brought into culture, fibroblast is the predominant cell type. In a preferred embodiment, the avian embryo cell preparation of the invention contains at least 80% fibroblasts, more preferably at least 90% fibroblasts, even more preferably between 95 and 100%.

In a particular embodiment, the method of the invention for preparing avian embryo cells further comprises the step of isolating fibroblasts from the filtered cells.

The cells may be maintained or cultivated or expanded in any suitable culture medium, preferably in a serum-free culture medium. Suitable culture media are commercially available and include, without limitation, EMEM, DMEM, M199, F12 and DME/F12.

Cells may be maintained in any suitable device, such as a flask, bottle, tube, ampoule, etc., typically under sterile conditions.

In this regard, a particular object of the invention resides in a culture or composition of avian embryo cells obtainable or obtained by a method as defined above, or progenitors of said cells.

The culture preferably comprises at least 90% fibroblasts.

As mentioned above, the invention is particularly suited for preparing embryo cells from chicken. Also, in a particular embodiment, the cells in the above culture or preparation are infected by a virus, such as a MDV, avian Reovirus, infectious bursal disease virus, Herpes virus of Turkeys, MDV- SB1 or MDV Rispens.

CEFs produced with whole embryos were compared against CEFs produced from embryo bodies-only so that we could clearly evaluate the whole process. In addition to confluency, survival and growth of whole embryo CEFs, we included virus growth curve studies, harvest time optimization and plant density impact.

We conducted studies on the growth/survival of the CEFs in the roller bottle and found that there was no difference between CEFs prepared from whole embryos versus bodies-only when it came to the number of cells we could recover from the bottle over a 3 day growth period.

An evaluation of the plant density and confluency was performed and showed that there was generally no difference between the CEF types in relation to confluency except a higher confluency produced from whole embryo CEFs at lower plant densities.

Also, we conducted full growth curves of virus growth with Marek's disease virus HVT-NDV. Our results show that CEFs produced from whole embryos were able to support strong growth of the viruses and to generate titers above minimum release and a yield above 5 ampoules/roller bottle.

These results therefore clearly show that the cells of the invention can be used to produce or expand viruses under conditions suitable for commercial use.

In a further aspect, the invention provides a method for producing or amplifying a virus, comprising infecting cells as defined above with a virus and, optionally, collecting the virus produced.

Infection can be made under any suitable conditions known per se in the art. For instance, cells can be infected in a culture medium with a virus preparation at a multiplicity of infection (MOI) of between 0.001 and 10. After production or amplification, the virus may be, preferably, inactivated. Inactivation can be performed with any technique known per se in the art such as chemical treatment with formaldehyde, BEI, BPL, etc.

In another aspect, the invention also relates to a method for producing a viral vaccine comprising (i) infecting cells as defined above with a virus under conditions allowing producing or amplification of the virus, (ii) optionally collecting the virus produced or amplified, (iii) optionally inactivating the virus produced or amplified in (i) or (ii), and (iv) formulating the virus produced or amplified as a vaccine composition.

In a particular embodiment of the method, the virus produced or amplified is collected, isolated, filtered at 20 microns or more, optionally inactivated, and formulated as a vaccine composition.

In another alternative embodiment of the method, the cells are filtered at 50 microns prior to step (i) and the cell supernatant or cell debris are used to produce the vaccine.

The virus may be any virus used for vaccine preparation in non-human or human medicine, such as preferably a MDV, avian Reovirus, infectious bursal disease virus, Herpes virus of Turkeys, MDV- SB1 or MDV Rispens.

The invention also relates to a vaccine composition comprising a virus obtained or obtainable by a method as disclosed above, and a suitable excipient or carrier.

Further aspects and advantages of the invention will be disclosed in the following experimental section, which shall be considered as illustrative.

### EXAMPLES

### Materials and Methods:

### Cell count

Manual cell counts were always performed with a hemocytometer on trypan blue stained cells. Automatic cell counts were performed with NC200 automatic cell counting machine. Samples for counting were typically diluted in the 10-100 fold range to reach the optimum counting range of the machine.

### Planting Bottles / Inoculation

Roller bottles were planted 24 hours before inoculation in different plant densities with Marek's Growth Media containing calf sera. Roller bottles were incubated at 37°C. The Marek's virus used in all studies was HVT-NDV x+4 orx+5.

### Harvesting

We harvested roller bottles two different ways. Non-infected roller bottles were harvested with pre-warmed (37°C) 0.25% Trypsin-EDTA. Each roller bottle received 50 mL and was incubated for 10 min at 37°C then cells decanted and centrifuged at 500 xg for 13 minutes. Infected roller bottles were harvested as in Marek's production with Trypsin-EDTA-Puck's buffer at a 1:1 mixture. Each roller received 100 mL (incubation temperature, duration and centrifugation were the same as in the case of the non-infected bottles).

### Batching / Filling

In those studies with infected roller bottles, the harvested material always was batched and filled in ampoules. Product was batched with 25% Cryoprotectant II and 75% Cryoprotectant I. Ampoules were filled manually, sealed with a Cozzoli Ampoule filler/sealer, and then frozen in Mr. Frosty containers. Ampoules were then transferred to liquid nitrogen the next day.

### Titration

Titration of the ampoules were performed on 100 mm tissue culture dishes. Generally, dishes were planted 24 hours before titration resulting in a confluent monolayer of CEFs. Ampoules were diluted 1 to 400 in Marek's diluents then further diluted as appropriate. Plates were inoculated and incubated in a CO₂ incubator for 5 days. Plates were stained with Gram crystal violet for 60 min at room temperature and then washed with warm tap water and left to air dry. Plaques were counted by microscope at 25x magnification.

### Statistics

Figures have error bars that represent 95% confidence intervals of the mean. Simplified, this means that if the measurement would be repeated, we would have a 95% chance of the result falling somewhere on the range of the bar. In the case of graphs with no bars, there we not enough replicates to generate the 95% interval. Regression lines (curves) were fit to the data where they could be applied and are indicated in the figure legends.

### Results:

### 1. Chicken Embryo cell production from whole embryos

CEFs were prepared from whole embryos by adding 150 embryos/flask and adding 1800ml room temperature trypsin/Puck's in a 1:1 ratio. Embryos are trypsinized in this fashion three times for 10 minutes each. Between each trypsinization, cells are decanted. Following trypsinization, cells are centrifuged at 450 x g for 10 minutes and supernatant discarded. Cell pellets are then combined and poured through gauze. The results of several experiments are presented below:
Bodies Only

| Exp | Cells/embryo |
|---|---|
| 1 | 3.20E+08 |
| 2 | 2.40E+08 |
| 3 | 2.40E+08 |
| 4 | 3.30E+08 |
| **total avg.** | **2.81E+08** |

Whole Embryos

| | |
|---|---|
| 1 | 6.80E+08 |
| 2 | 4.86E+08 |
| 3 | 5.50E+08 |
| 4 | 6.30E+08 |
| **total avg.** | **5.99E+08** |

### Effect of temperature and embryo number

A comparative experiment was performed to further improve the process conditions and yield. More particularly, the temperature was tested, as well as the number of embryos per flask. The results are show in the table below. Room temperature means 20-25°C.

| **Embryos / Flask** | **Trypsin Temperature** | **Volume (mL)** | **Average Cell Count/ Viability** | **Cells / Embryo** |
|---|---|---|---|---|
| 100 | RT | 1270 | 5.60E+07/ 67% | 7.11E+08 |
| 150 | RT | 1269 | 7.89E+07/ 82% | 6.67E+08 |
| 150 | 37°C | 1335 | 7.61E+07/ 84% | 6.77E+08 |
| 200 | RT | 1340 | 8.20E+07/ 80% | 5.97E+08 |

### 2. Survival studies

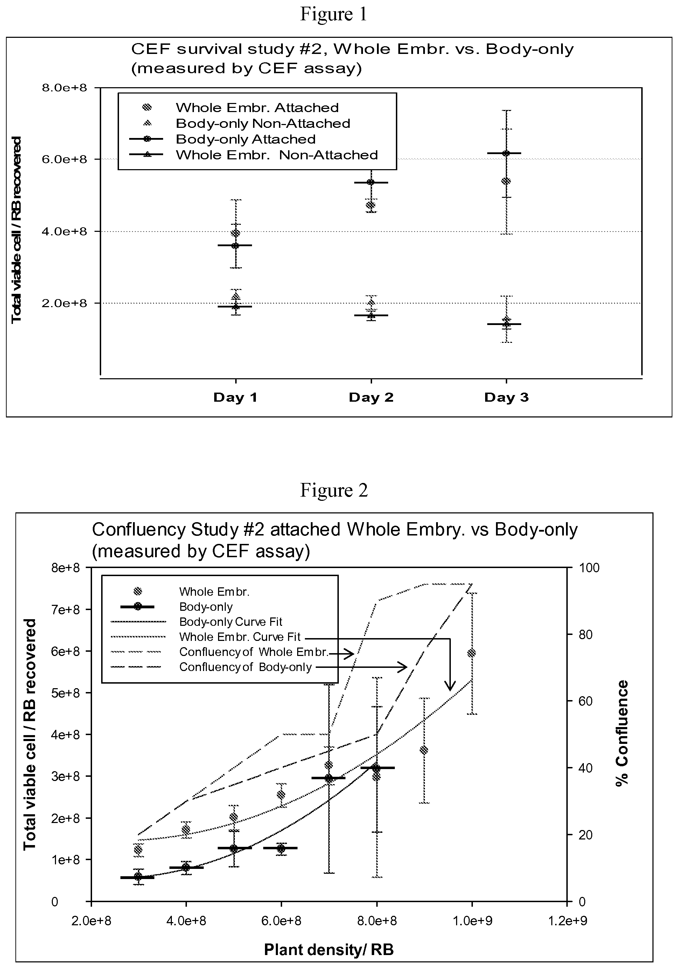

As a beginning point, we compared the survival rate of the CEFs produced from full embryos versus CEFs from bodies-only. We planted roller bottles at the same cell density per roller bottle with both cell types and monitored them over 3 days for cell growth and survival. Cell counts were performed on attached and unattached cells from five roller bottles at each time point. Figures 1 summarizes these results. We observed a suitable growth and survival of the CEF cells obtained from Whole embryos with no significant difference between the two.

### 3. Confluency studies

The aim of this study was to evaluate the possible alteration of proliferation kinetics of the whole embryo CEFs. In this experiment, we planted roller bottles with CEF cells at plant densities from 3.0E+08 to 1.0E+09 and checked the cell counts of the attached cells after 24 hours to determine the lowest plant density which results in a confluent monolayer for each CEF type (whole embryo or bodies-only). Five roller bottles were planted at each density. Confluency was read by three different technicians and averaged. The results are presented in Figure 2 and show that there is no significant difference between the CEF types when it comes to recovered cells from the roller bottles. The results also show that whole embryo-derived CEFs produced a slightly better confluency at lower plant densities.

### 4. Growth curve studies

In these studies our aim was to clarify the possible alterations in the time course of the HVT infection in the different CEFs or, in other words, to evaluate the time distribution of the virus proliferation. Bottles were planted at the same density and infected with a similar MOI. Roller bottles were harvested at multiple time points post infection as indicated. Ampoules were batched, filled and titered as described previously.

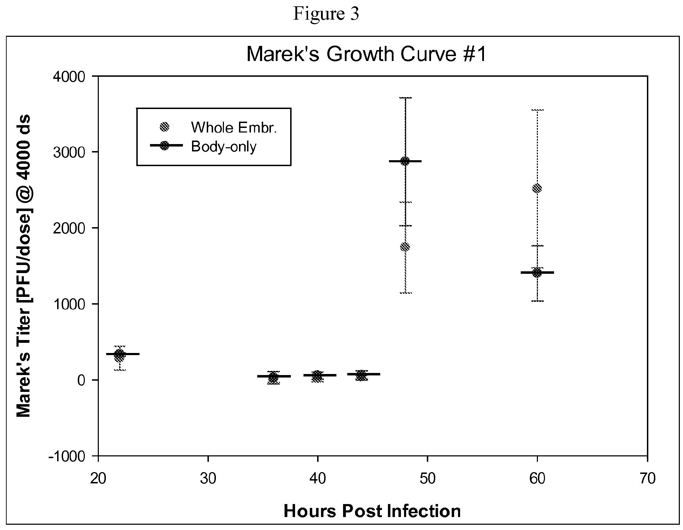

Figure 3 shows the result from the growth curve study and indicates that both the bodies-only and the whole embryos produced approximately the same titer. The bodies-only embryo curve seemed to come up faster, and both peaked near 3000 pfu/ds.

### 5. Filtration Trials

In order to protect the cosmetic appearance of the final product and to realize the gain that processing the whole embryo imparts, filtration was performed to remove the 'black specs' that originate from the eyes of the embryos. To this end, we performed two filtration trials on concentrated CEFs prepared from the whole embryos. Concentrated in this case means that the CEFs were filtered at a density which can be expected after processing. This is much higher than the plant density. Typically, this density from bodies-only embryos would be approximately 2-3E+07 cells/mL. For these studies we filtered cells at a density of 7E+07cells/mL to account for the increased number of cells from whole embryos. The filter we tested was a 50 micron polypropylene depth filter from Sartorius. Two trials were performed, a small scale (0.5 L CEFs using a disc filter version of the 50 micron PP filter ), and an intermediate scale (11.5 L CEFs using a size 9 version of the 50 micron PP filter). During the filtration, we recorded the cell counts of the filtrate and the viability of the cells.

The results from the small scale trial show that the viability held at ~80% and the cell count fluctuated somewhat but remained steady throughout. At the end of the filtration, we counted the total volume of filtrate and found the overall loss to be 12%.

In the intermediate scale trial, the viability held at ~80% throughout, however the cell count was falling steadily. At the end of the trial we counted the filtrate and found overall a 20% loss.

To avoid the loss of cells, we designed a system to backflush the filter. CEFs were counted and then filtered through a 50 micron polypropylene filter (Sartorius). The backflush system was rigged and the filter rinsed in reverse. No cells were lost in the filtration using this backflush system, as shown in the Table below.

| **phase** | volume (ml) | total cell count |
|---|---|---|
| **pre filter** | 5000 | 2.14E+11 |
| **post filter** | 5000 | 2.05E+11 |
| **after back flush** | 9000 | 2.41E+11 |

### 7. Pilot study

Taking into consideration the results from all the preceding studies, a pilot size batch of Marek's virus and Reovirus was produced.

### Reovirus' production

A small scale batch of avian Reovirus strain S1133 was prepared to illustrate how whole embryos can be used to produce virus. 300 whole embryos were prepared to CEF cells as described previously. Roller bottles were seeded at 1.4E+09 CEFs per bottle and co-infected with Reovirus. After 4 days, bottles were harvested and the supernatant pooled. Samples were taken for live virus titration prior to inactivation. The results are presented in the table below.

| CEF | |
|---|---|
| No. of embryos | 300 |
| Average weight of embryos (g) | 3,2 |
| Volume of CEF (ml) | 4660 |
| Live cell count/ml | 4,00E+07 |
| Cells/embryo | 6,21E+08 |
| Virus Titer (TCID50) | 8.6, 8.5 |

### Marek's production

A small scale trial of Marek's disease virus vaccine comprised of HVT-ILT virus was prepared using whole embryos. CEF cells were prepared from whole embryos as described previously and seeded into roller bottles at 8.0E+08 cells per bottle. Bottles were infected at 24 hours post plant with virus using and MOI of 0.006. After 48 hours, bottles were harvested by trypsinizing and decanting. Harvested cells were centrifuged, batched and filled into ampoules.

The results from the pilot batch are presented in the table below

| CEF | |
|---|---|
| No. of embryos | 150 |
| Volume of CEF (ml) | 1600 |
| Live cell count/ml | 3,45E+07 |
| Cells/embryo | 3,68E+08 |
| Virus Titer (pfu/ds) | 4280/4640 |

## Claims

1. A method for preparing avian embryo cells comprising:
a) providing whole embryos obtained from avian eggs, each of said whole avian embryos having a head and eyes,
b) placing said whole avian embryos into one or several suitable recipients, such that each of said recipients contain between 50 and 150 whole avian embryos,
c) preparing avian embryo cells by enzymatic treatment of the whole embryos at a temperature comprised between 20°C and 40°C, and
d) filtering the cells of step c) with a filter having a pore size of between 20 microns and 50 microns.

2. The method of claim 1, wherein the avian egg is a Specified Pathogen Free avian egg.

3. The method of claim 1, wherein the avian embryo cells are cultured or expanded prior to or after filtration step d).

4. The method of claim 1, wherein the avian embryo cells are infected by a virus prior to or after filtration step d).

5. The method of claim 1, wherein the enzymatic treatment is trypsinization.

6. The method of any one of the preceding claims, wherein the avian embryo cells are fibroblasts.

7. The method of any one of the preceding claims, wherein the avian is chicken.

8. The method of any one of the preceding claims, wherein the cells are maintained in a serum-free culture medium.

9. A culture or composition of avian embryo cells obtainable by the method of claim 1.

10. The culture or composition of claim 9, wherein said avian embryo cells are chicken embryo fibroblasts.

11. The culture or composition of claim 9, wherein the cells are infected by a virus.

12. A method for producing or amplifying a virus, comprising infecting cells of claim 9 with a virus, optionally collecting the virus produced and, optionally inactivating the virus.

13. The method of claim 12, wherein the virus is a MDV.

14. A method for producing a viral vaccine comprising (i) infecting cells of claim 9 with a virus under conditions allowing producing or amplification of the virus, (ii) optionally collecting the virus produced or amplified, (iii) optionally inactivating the virus produced or amplified in (i) or (ii), and (iv) formulating the virus produced or amplified as a vaccine composition.

15. The method of claim 14, wherein the virus is a MDV.

16. A vaccine composition comprising the virus obtained by the method of claim 12 and a suitable excipient or carrier.

## Patentansprüche

1. Verfahren zur Herstellung von Vogelembryozellen, umfassend:
a) Bereitstellen ganzer Embryonen, die aus Vogeleiern erhalten werden, wobei jeder dieser ganzen Vogelembryonen einen Kopf und Augen aufweist,
b) Platzieren der ganzen Vogelembryonen in einen oder mehrere geeignete Empfänger, derart dass jeder der Empfänger zwischen 50 und 150 ganze Vogelembryonen enthält,
c) Herstellen von Vogelembryozellen durch enzymatische Behandlung der ganzen Embryonen bei einer Temperatur zwischen 20 °C und 40 °C und
d) Filtrieren der Zellen aus Schritt c) mit einem Filter mit einer Porengröße zwischen 20 Mikrometern und 50 Mikrometern.

2. Verfahren nach Anspruch 1, wobei das Vogelei ein spezifiziert pathogenfreies Vogelei ist.

3. Verfahren nach Anspruch 1, wobei die Vogelembryozellen vor oder nach dem Filtrationsschritt d) kultiviert oder expandiert werden.

4. Verfahren nach Anspruch 1, wobei die Vogelembryozellen vor oder nach dem Filtrationsschritt d) mit einem Virus infiziert werden.

5. Verfahren nach Anspruch 1, wobei die enzymatische Behandlung eine Trypsinierung ist.

6. Verfahren nach einem der vorstehenden Ansprüche, wobei die Vogelembryozellen Fibroblasten sind.

7. Verfahren nach einem der vorstehenden Ansprüche, wobei der Vogel ein Küken ist.

8. Verfahren nach einem der vorstehenden Ansprüche, wobei die Zellen in einem serumfreien Kulturmedium gehalten werden.

9. Kultur oder Zusammensetzung aus Vogelembryozellen, die durch das Verfahren nach Anspruch 1 erhältlich sind.

10. Kultur oder Zusammensetzung nach Anspruch 9, wobei die Vogelembryozellen Kükenembryofibroblasten sind.

11. Kultur oder Zusammensetzung nach Anspruch 9, wobei die Zellen mit einem Virus infiziert sind.

12. Verfahren zur Herstellung oder Amplifikation eines Virus, umfassend Infizieren von Zellen nach Anspruch 9 mit einem Virus, gegebenenfalls Sammeln des hergestellten Virus und gegebenenfalls Inaktivieren des Virus.

13. Verfahren nach Anspruch 12, wobei das Virus ein MDV ist.

14. Verfahren zur Herstellung eines viralen Impfstoffs, umfassend (i) Infizieren von Zellen nach Anspruch 9 mit einem Virus unter Bedingungen, die die Herstellung oder Amplifikation des Virus ermöglichen, (ii) gegebenenfalls Sammeln des hergestellten oder amplifizierten Virus, (iii) gegebenenfalls Inaktivieren des in (i) oder (ii) hergestellten oder amplifizierten Virus und (iv) Formulieren des hergestellten oder amplifizierten Virus als Impfstoffzusammensetzung.

15. Verfahren nach Anspruch 14, wobei das Virus ein MDV ist.

16. Impfstoffzusammensetzung, umfassend das durch das Verfahren nach Anspruch 12 erhaltene Virus und einen geeigneten Hilfsstoff oder Träger.

## Revendications

1. Procédé de préparation de cellules d'embryons aviaires comprenant :
a) la fourniture d'embryons entiers obtenus à partir d'œufs d'oiseaux, chacun de ces embryons aviaires entiers ayant une tête et des yeux,
b) le placement desdits embryons aviaires entiers dans un ou plusieurs récipients appropriés, de sorte que chacun desdits récipients contienne entre 50 et 150 embryons aviaires entiers,
c) la préparation de cellules d'embryons aviaires par traitement enzymatique des embryons entiers à une température comprise entre 20 °C et 40 °C, et
d) le filtrage des cellules de l'étape c) à l'aide d'un filtre dont la taille des pores est comprise entre 20 et 50 microns.

2. Procédé selon la revendication 1, dans lequel l'œuf aviaire est un œuf aviaire exempt de pathogènes spécifiés.

3. Procédé selon la revendication 1, dans lequel les cellules d'embryons aviaires sont cultivées ou étendues avant ou après l'étape de filtration d).

4. Procédé selon la revendication 1, dans lequel les cellules d'embryons aviaires sont infectées par un virus avant ou après l'étape de filtration d).

5. Procédé selon la revendication 1, dans lequel le traitement enzymatique est la trypsinisation.

6. Procédé selon l'une quelconque des revendications précédentes, dans lequel les cellules d'embryons aviaires sont des fibroblastes.

7. Procédé selon l'une quelconque des revendications précédentes, dans lequel l'oiseau est un poulet.

8. Procédé selon l'une quelconque des revendications précédentes, dans lequel les cellules sont maintenues dans un milieu de culture sans sérum.

9. Culture ou composition de cellules d'embryons aviaires obtenue par le procédé selon la revendication 1.

10. Culture ou composition selon la revendication 9, dans laquelle les cellules d'embryons aviaires sont des fibroblastes d'embryons de poulet.

11. Culture ou composition selon la revendication 9, dans laquelle les cellules sont infectées par un virus.

12. Procédé de production ou d'amplification d'un virus, comprenant l'infection de cellules selon la revendication 9 avec un virus, éventuellement la collecte du virus produit et, éventuellement, l'inactivation du virus.

13. Procédé selon la revendication 12, dans lequel le virus est un MDV.

14. Procédé de production d'un vaccin viral comprenant (i) l'infection de cellules selon la revendication 9 par un virus dans des conditions permettant la production ou l'amplification du virus, (ii) éventuellement la collecte du virus produit ou amplifié, (iii) éventuellement l'inactivation du virus produit ou amplifié en (i) ou (ii), et (iv) la formulation du virus produit ou amplifié en tant que composition vaccinale.

15. Procédé selon la revendication 14, dans lequel le virus est un MDV.

16. Composition vaccinale comprenant le virus obtenu par le procédé selon la revendication 12 et un excipient ou support approprié.
